# EUROPÄISCHE PATENTSCHRIFT

(11) **EP 2 079 718 B1**
(45) Veröffentlichungstag und Bekanntmachung des Hinweises auf die Patenterteilung: **07.04.2010**
(21) Anmeldenummer: 07803420.4
(22) Anmeldetag: 12.09.2007
(51) Int. Cl.: C07D 295/02

(54) **VERFAHREN ZUR KONTINUIERLICHEN DESTILLATIVEN AUFTRENNUNG VON GEMISCHEN ENTHALTEND MORPHOLIN (MO), MONOAMINODIGLYKOL (ADG), AMMONIAK UND WASSER**
METHOD FOR THE CONTINUOUS SEPARATION OF MIXTURES COMPRISING MORPHOLINE (MO), MONOAMINODIGLYCOL (ADG), AMMONIA, AND WATER BY MEANS OF DISTILLATION
PROCÉDÉ DE SÉPARATION PAR DISTILLATION EN CONTINU DE MÉLANGES CONTENANT DE LA MORPHOLINE (MO), DU MONOAMINODIGLYCOL (ADG), DE L'AMMONIAC ET DE L'EAU

(30) Priorität: 28.09.2006 EP 06121427
(43) Veröffentlichungstag der Anmeldung: 22.07.2009
(73) Patentinhaber: BASF SE, 67056 Ludwigshafen (DE)
(72) Erfinder: SCHMIDTKE Helmut, 64625 Bensheim (DE); BUSSMANN Oliver, 67071 Ludwigshafen (DE); VERSCH Ralph, 55234 Eppelsheim (DE); RHEUDE Udo, 67166 Otterstadt (DE); LEYK Uwe, 67551 Worms (DE); JULIUS Manfred, 67117 Limburgerhof (DE); RUDLOFF Martin, 67161 Gönnheim (DE); HENKES Erhard, 64683 Einhausen (DE)
(86) Internationale Anmeldenummer: PCT/EP2007/059556
(87) Internationale Veröffentlichungsnummer: WO 2008/037589

(56) Entgegenhaltungen:
- EP-A- 0 696 572
- EP-A- 0 963 975
- US-A- 3 154 544
- US-A- 3 155 657

## Beschreibung

Die vorliegende Erfindung betrifft ein Verfahren zur kontinuierlichen destillativen Auftrennung von Gemischen enthaltend Morpholin (MO), Monoaminodiglykol (ADG), Ammoniak und Wasser, erhalten durch Umsetzung von Diethylenglykol (DEG) der Formel 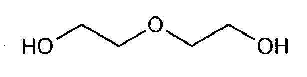 mit Ammoniak.
Aminodiglykol (ADG) [= 2-(2-Aminoethoxy)ethanol = 2,2'-Aminoethoxyethanol, Formel 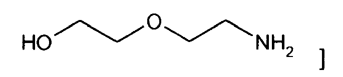 und Morpholin finden u.a. Verwendung als Lösungsmittel, Stabilisatoren, zur Synthese von Chelat-Bildnern, Kunstharzen, Arzneimitteln, Inhibitoren und grenzflächenaktiven Substanzen.
N-Ethyl-morpholin (E-MO) wird u.a. als Katalysator zur Herstellung von Polyurethanschäumen verwendet.

Zur Herstellung von ADG und Morpholin sind in der Literatur zahlreiche Verfahren beschrieben.

Ullmann's Encyclopedia of Industrial Chemistry, 6th Edition, 2000 electronic release, Wiley-VCH Verlag, Rubrik 'cydic amines' im Kapitel 'aliphatic amines' beschreibt die Synthese von ADG und MO durch Aminierung von DEG unter Wasserstoffdruck und in gegenwart eines Kobalt- oder Nickelkatalysators (Zitate: EP-A-696 572 (BASF AG), DE-A-1 049 864) oder anderer Katalysatoren (Zitate: DE-A-3 002 342, DE-A-3 125 662 (BASF AG), US 3 155 657).

Die ältere deutsche Patentanmeldung Nr. 102005047458.6 vom 30.09.05 und die ältere europäische (Folge)Patentanmeldung Nr. 06101339.7 vom 06.02.06 (BASF AG) betreffen ein Verfahren zur Herstellung von ADG und Morpholin durch Umsetzung von DEG mit Ammoniak in Gegenwart eines spezifischen Kupfer, Nickel und Kobalt - Heterogenkatalysators sowie allgemein die Aufarbeitung durch mehrstufige Destillation.

Die Patentschrift US 3,155,657 beschreibt ein kontinuierliches verfahren zur Aufarbeitung der Reaktionsgemische von Ammoniak und Diethylenglykol mittels mehrstufiger Destillation.

Zwei parallele europäische Patentanmeldungen mit gleichem Anmeldetag (beide BASF AG) betreffen Verfahren zur kontinuierlichen destillativen Auftrennung von Gemischen enthaltend Morpholin (MO), Monoaminodiglykol (ADG), Ammoniak und Wasser, erhalten durch Umsetzung von Diethylenglykol (DEG) mit Ammoniak.

Eine parallele europäische Patentanmeldung mit gleichem Anmeldetag (BASF AG) betrifft ein Verfahren zur Herstellung von ADG in Elektronik-Qualität.

Die Morpholin- und Monoaminodiglykolsynthese ist gekennzeichnet durch die Bildung von einer Vielzahl an Nebenkomponenten. Die Abtrennung der nicht umgesetzten Einsatzstoffe, Wert- sowie der Nebenprodukte erfolgt destillativ was zu einem erheblichen apparativen Aufwand führt. Außer den Hochsiedern werden alle Stoffe ein- oder sogar mehrfach über Kopf abgetrennt. Ein deutlicher Energieaufwand ist die Folge.

Der vorliegenden Erfindung lag die Aufgabe zugrunde, unter Überwindung eines Nachteils oder mehrerer Nachteile des Stands der Technik, ein verbessertes wirtschaftliches Verfahren zur Auftrennung von Gemischen enthaltend Morpholin (MO), Monoaminodiglykol (ADG), Ammoniak und Wasser und ggf. N-Ethyl-morpholin (E-MO) und ggf. 1,2-Ethylendiamin (EDA) und ggf. organische Produkte mit einem Siedepunkt > 224,8°C (1,013 bar) aufzufinden. Die einzelnen organischen Komponenten (Amine), insbesondere MO und ADG und ggf. E-MO, sollten dabei in hoher Reinheit und Qualität (z.B. Farbqualität) anfallen.

Demgemäß wurde ein Verfahren zur kontinuierlichen destillativen Auftrennung von Gemischen enthaltend Morpholin (MO), Monoaminodiglykol (ADG), Ammoniak und Wasser, erhalten durch Umsetzung von Diethylenglykol (DEG) mit Ammoniak, gefunden, welches dadurch gekennzeichnet ist, dass
in einer ersten Destillationskolonne K10 Ammoniak über Kopf abgetrennt wird,
der Sumpfaustrag von K10 einer zweiten Destillationskolonne K20 zugeführt wird, in der Wasser und organische Produkte über Kopf bei einer Kopftemperatur im Bereich von 45 bis 198 °C und einem Druck im Bereich von 0,1 bis 15 bar abgetrennt werden, der Sumpfaustrag von K20 einer dritten Destillationskolonne K30 zugeführt wird, in der MO und organische Produkte mit einem Siedepunkt < 140 °C (1,013 bar) über Kopf oder in einem Seitenabzug abgetrennt werden und ADG und organische Produkte mit einem Siedepunkt > 190 °C (1,013 bar) über Sumpf abgetrennt werden,
der MO enthaltende Strom, der bei der Kolonne K30 über Kopf oder im Seitenabzug abgetrennt wird, einer vierten Kolonne K40 zugeführt wird, in der organische Produkte mit einem Siedepunkt ≤ 128 °C (1,013 bar), bevorzugt < 128 °C (1,013 bar), über Kopf und MO und organische Produkte mit einem Siedepunkt ≥ 128 °C (1,013 bar) über Sumpf abgetrennt werden, und
der Sumpfaustrag von K40 einer fünften Destillationskolonne K55 zugeführt wird, in der MO über Kopf und organische Produkte mit einem Siedepunkt ≥ 128 °C (1,013 bar) über Sumpf abgetrennt werden.

Die in Kolonne K55 über Sumpf abgetrennten Produkte werden ganz oder teilweise, bevorzugt ganz, in den Zulauf zur Kolonne K30 zurückgeführt.

Die Kolonne K10 weist bevorzugt im Bereich von 3 bis 30, besonders im Bereich von 5 bis 20, theoretische Trennstufen auf.
Sie wird bevorzugt bei einem Druck im Bereich von 5 bis 30 bar, besonders 10 bis 20 bar, betrieben.

Bevorzugt befindet sich die Zulaufstelle bei Kolonne K10 im oberen Drittel, bezogen auf die Zahl der theoretischen Trennstufen.

Die Kolonne K20 weist bevorzugt im Bereich von 25 bis 70, besonders im Bereich von 30 bis 60, theoretische Trennstufen auf.
Sie wird bevorzugt bei einem Druck im Bereich von 0,1 bis 10 bar, besonders 0,8 bis 7 bar, betrieben.

Bevorzugt befindet sich die Zulaufstelle bei Kolonne K20 im mittleren Drittel, bezogen auf die Zahl der theoretischen Trennstufen.

In der Kolonne K20 wird bevorzugt Waser abgetrennt. Mit diesem Wasser werden bevorzugt organische Produkte als Minimumazeotrop über Kopf abgetrennt, welche zum Teil höhere Siedepunkte als das Sumpfprodukt Morpholin haben.

Die Kolonne K30 weist bevorzugt im Bereich von 5 bis 25, besonders im Bereich von 7 bis 20, theoretische Trennstufen auf.
Sie wird bevorzugt bei einem Druck im Bereich von 0,01 bis 5 bar, besonders 0,05 bis 2,5 bar, betrieben.

Bevorzugt befindet sich die Zulaufstelle bei Kolonne K30 im oberen Drittel, bezogen auf die Zahl der theoretischen Trennstufen.
In der alternativen, weniger bevorzugten Ausführungsform befindet sich der Seitenabzug bevorzugt 1 bis 8 theoretische Trennstufen, besonders 2 bis 6 theoretische Trennstufen, oberhalb der Zulaufstelle.

Die Kolonne K40 weist bevorzugt im Bereich von 10 bis 80, besonders im Bereich von 15 bis 60, theoretische Trennstufen auf.
Sie wird bevorzugt bei einem Druck im Bereich von 0,01 bis 12 bar, besonders 0,5 bis 6 bar, betrieben.

Bevorzugt befindet sich die Zulaufstelle bei Kolonne K40 im mittleren oder unteren, besonders mittleren, Drittel, bezogen auf die Zahl der theoretischen Trennstufen.

In der Kolonne K40 werden organische Produkte mit einem Siedepunkt ≤ 128°C (1,013 bar) (Leichtsieder = LS), wie z.B. EDA, über Kopf abgetrennt und MO und organische Produkte mit einem Siedepunkt ≥ 128 °C (1,013 bar) über Sumpf abgetrennt.

Die in der Kolonne K40 über Kopf abgetrennten organischen Produkte, insbesondere EDA, können vorteilhaft ganz oder teilweise in den Zulauf zur Kolonne K20 zurückgeführt werden.

In einer weiteren Ausführungsform kann durch weitere destillative Aufreinigung des Kopfdestillats der K40 reines EDA als Wertprodukt gewonnen werden.

Die Kolonne K55 weist bevorzugt im Bereich von 1 bis 15, besonders im Bereich von 2 bis 12, theoretische Trennstufen auf.
Sie wird bevorzugt bei einem Druck im Bereich von 0,01 bis 12 bar, besonders 0,5 bis 6 bar, betrieben.

Bevorzugt befindet sich die Zulaufstelle bei Kolonne K55 im unteren oder mittleren, besonders mittleren, Drittel, bezogen auf die Zahl der theoretischen Trennstufen.

In der Kolonne K55 wird MO über Kopf und werden organische Produkte mit einem Siedepunkt ≥ 128 °C (1,013 bar) über Sumpf abgetrennt.

In einer besonderen Ausführungsform wird der wasser- und organische Produkte enthaltende Strom, der bei der Kolonne K20 über Kopf abgetrennt wird, einer Kolonne K50 zugeführt, in der wässrige N-Ethyl-morpholin-Lösung (wässrige E-MO-Lösung) über Kopf oder einen flüssigen Seitenabzug, wobei sich der flüssige Seitenabzug bevorzugt im oberen Drittel der Kolonne, bezogen auf die Zahl der theoretischen Trennstufen, befindet, und Wasser über Sumpf abgetrennt wird.

Die Kolonne K50 weist bevorzugt im Bereich von 10 bis 50, besonders im Bereich von 15 bis 40, theoretischen Trennstufen auf.
Sie wird bevorzugt bei einem Druck im Bereich von 0,1 bis 16 bar, besonders 0,2 bis 8 bar, betrieben.

Bevorzugt befindet sich die Zulaufstelle bei Kolonne K50 im oberen oder mittleren, besonders im mittleren, Drittel, bezogen auf die Zahl der theoretischen Trennstufen.

Die wässrige N-Ethyl-morpholin-Lösung wird zur Gewinnung des reinen E-MOs zunächst entwässert. Als entwässerndes Mittel wird bevorzugt Natronlauge, z.B. als 40-60 Gew.-%ige wässrige Lösung, besonders 50 Gew.-%ige wässrige Lösung, eingesetzt. Bevorzugt wird die Entwässerung mit der Natronlauge kontinuierlich in einer Extraktionskolonne durchgeführt. Die Extraktionstemperatur liegt bevorzugt zwischen 25-60°C, besonders zwischen 30-55 °C. Die Natronlauge wird dabei auf 15-35 Gew.-%, besonders 20-30 Gew.-%, verdünnt.
Nach der Phasentrennung wird die organische Phase in einer kontinuierlichen oder diskontinuierlichen Destillation aufgearbeitet. Die Destillation wird bevorzugt diskontinuierlich in einer Destillationsblase durchgeführt.
Die Kopfprodukte fallen dabei nacheinander an: ggf. Ethylamin, ggf. Ethanol als wässriges Azeotrop, ggf. N-Methyl-morpholin als wässriges Azeotrop, ggf. wasserfreies N-Methyl-morpholin und das Wertprodukt N-Ethyl-morpholin (E-MO).

In einer bevorzugten Ausführungsform wird der Sumpfaustrag von K30 einer Destillationskolonne K60 zugeführt, in der organische Produkte mit einem Siedepunkt
≤ 224,8°C (1,013 bar) über Kopf und ADG und organische Produkte mit einem Siedepunkt ≥ 224,8 °C (1,013 bar) über Sumpf abgetrennt werden.

Die Kolonne K60 weist bevorzugt im Bereich von 5 bis 40, besonders im Bereich von 10 bis 30, theoretische Trennstufen auf.
Sie wird bevorzugt bei einem Druck im Bereich von 0,005 bis 1 bar, besonders 0,05 bis 0,8 bar, betrieben.

Bevorzugt befindet sich die Zulaufstelle bei Kolonne K60 im mittleren oder oberen, besonders mittleren, Drittel, bezogen auf die Zahl der theoretischen Trennstufen.

In einer bevorzugten Ausführungsform werden in der Kolonne K60 über Kopf abgetrennte organische Produkte, wie z.B. N-(2-Aminoethyl)morpholin, 2-(2-Aminoethoxy)-ethylamin, in die Umsetzung von DEG mit Ammoniak zurückgeführt.

Um Aufpegelungen einzelner Komponenten im Kreislauf der Produktionsanlage zu vermeiden, wird bevorzugt ein Teilstrom des im Kolonnenkopf abgetrennten Destillates ausgeschleust. Der Anteil des zurückgeführten Stromes beträgt bevorzugt 40-100 Gew.-%, besonders bevorzugt 50-100 Gew.-%, des im Kolonnenkopf abgetrennten Destillates.

Der ADG enthaltende Strom, der bei der Kolonne K60 im Sumpfabzug abgetrennt wird, wird bevorzugt einer Kolonne K70 zugeführt, in der ADG über Kopf und DEG und organische Produkte mit einem Siedepunkt ≥ 255 °C (1,013 bar) über Sumpf abgetrennt werden.

Die Kolonne K70 weist bevorzugt im Bereich von 10 bis 60, besonders im Bereich von 20 bis 50, theoretische Trennstufen auf.
Sie wird bevorzugt bei einem Druck im Bereich von 0,005 bis 1 bar, besonders 0,01 bis 0,8 bar, betrieben.

Bevorzugt befindet sich die Zulaufstelle bei Kolonne K70 im oberen oder mittleren, bevorzugt oberen, Drittel, bezogen auf die Zahl der theoretischen Trennstufen.

In Kolonne K70 über Sumpf abgetrennte Produkte sind DEG und ggf. Morpholylaminodiglykol, Morpholinodiglykol.
(Morpholylaminodiglykol = 4-(2-(2-Aminoethoxy)-ethyl)-morpholin, C₈H₁₈N₂O₂;
Morpholinodiglykol (Morpholinylethoxyethanol) CAS-Nr. 3603-45-0, C₈H₁₇NO₃)

Der DEG enthaltende Strom, der bei der Kolonne K70 im Sumpfabzug abgetrennt wird, wird bevorzugt einer Kolonne K80 zugeführt, in der DEG über Kopf und organische Produkte mit einem Siedepunkt ≥ 255 °C (1,013 bar) (Hochsieder = HS) über Sumpf abgetrennt werden.

Die Kolonne K80 weist bevorzugt im Bereich von 10 bis 60, besonders im Bereich von 20 bis 50, theoretische Trennstufen auf.
Sie wird bevorzugt bei einem Druck im Bereich von 0,005 bis 1 bar, besonders 0,05 bis 0,8 bar, betrieben.

Bevorzugt befindet sich die Zulaufstelle bei Kolonne K80 im oberen oder mittleren, bevorzugt mittleren, Drittel, bezogen auf die Zahl der theoretischen Trennstufen.

Bevorzugt werden in Kolonne K80 über Kopf abgetrennte Produkte, wie z.B. DEG, Morpholylaminodiglykol, in die Umsetzung von DEG mit Ammoniak zurückgeführt.

Der Anteil des zurückgeführten Stromes beträgt bevorzugt 80-100 Gew.-%, besonders bevorzugt 95-100 Gew.-%, des im Kolonnenkopf abgetrennten Destillates.

Das erfindungsgemäße Verfahren ist in besonderen Ausführungsformen durch folgende Wärmeintegrationsmaßmahmen zusätzlich vorteilhaft:

Die Wärme aus den Brüden der K80 kann in der K20, K50, K30, K40, K55 und/oder K60 integriert werden, bevorzugt in der K50, K30, K55 und/oder K60.

Die Wärme aus den Brüden der K70 kann in der K20, K50, K30, K40, K55 und/oder K60 integriert werden, bevorzugt in der K20 und/oder K40.

Die Wärme aus den Brüden der K55 kann in der K20, K50, K30, K40 und/oder K60 integriert werden, bevorzugt in der K50, K30 und/oder K60.

Die Wärme aus den Brüden der K40 kann in der K20, K50, K30, K55 und/oder K60 integriert werden, bevorzugt in der K50.

Diese Wärmeintegration kann wie folgt ausgeführt werden:
Um die anfallende Wärme der Brüden maximal nutzen zu können, wird bevorzugt auf ein Wärmeträgermedium verzichtet und die Brüdenströme werden bevorzugt direkt in den entsprechenden Verdampfern, anstatt des Heizdampfes, kondensiert. Als Verdampfer können jeweils Naturumlaufverdampfer, Zwangsumlaufverdampfer, Zwangsumlaufentspannungsverdampfer, Fallfilmverdampfer, Robertverdampfer, Kettle-Type-Verdampfer, Dünnschichtverdampfer oder Kletterfilmverdampfer eingesetzt werden. Bevorzugt werden jeweils Naturumlaufverdampfer, Zwangsumlaufverdampfer, Zwangsumlaufentspannungsverdampfer, Fallfilmverdampfer, Robertverdampfer oder Kettle-Type-Verdampfer eingesetzt.
Die Restbrüden werden bevorzugt jeweils in einem Nachkondensator verflüssigt.

Weiterhin ist es vorteilhaft, die Reaktionswärme aus der Synthese des aufzutrennenden Gemisches abzuführen, insbesondere über Siedekühlung (Wasserdampf), und in der Destillation zu integrieren. Bei der Synthese sind insbesondere die Ausführungsformen (A) und (B) bevorzugt.

Die Reaktionswärme kann hierbei in den Kolonnen K20, K50, K30, K40, K55, K60, K70 und/oder K80, bevorzugt in den Kolonnen K20, K50, K30, K55 und/oder K60 integriert werden.

In einer weiteren besonders bevorzugten Ausführungsform wird der Sumpfaustrag von K30 einer Destillationskolonne K60 zugeführt, wobei es sich bei der Kolonne K60 um eine Trennwandkolonne (TK) handelt.

Die Trennwandkolonne (TK) weist bevorzugt eine Trennwand (T) in Kolonnenlängsrichtung unter Ausbildung eines oberen gemeinsamen Kolonnenbereichs (1), eines unteren gemeinsamen Kolonnenbereichs (6), eines Zulaufteils (2, 4) mit Verstärkungsteil (2) und Abtriebsteil (4), sowie eines Entnahmeteils (3, 5) mit Verstärkungsteil (3) und Abtriebsteil (5) auf, wobei die Zuführung des Sumpfaustrags von K30 im oberen oder mittleren Drittel, besonders oberen Drittel, des Zulaufteils (2, 4), bezogen auf die Zahl der theoretischen Trennstufen des Zulaufteils, die Abführung von organischen Produkten mit einem Siedepunkt > 255 °C (1,013 bar) über Sumpf, die Abführung von organischen Produkten mit einem Siedepunkt ≤ 224,8 °C (1,013 bar) über Kopf, die Abführung von ADG aus dem Kolonnenbereich 1 und optional, in einer besonderen Ausführungsform bevorzugt, die Abführung von dampfförmigen organischen Produkten mit einem Siedepunkt ≥ 224,8 °C (1,013 bar), besonders > 235 °C (1,013 bar), wie z.B. DEG, aus dem oberen oder mittleren Drittel, besonders oberen Drittel, des Entnahmeteils (3, 5) (Seitenabzug), bezogen auf die Zahl der theoretischen Trennstufen des Entnahmeteils, erfolgt.

In der Kolonne K60 über den dampfförmigen Seitenabzug abgetrennte organische Produkte, wie z.B. DEG, werden bevorzugt in die Umsetzung von DEG mit Ammoniak zurückgeführt.

In einer weiteren vorteilhaften Ausführungsform weist die Trennwandkolonne (TK) eine Trennwand (T) in Kolonnenlängsrichtung unter Ausbildung eines oberen gemeinsamen Kolonnenbereichs (1) und (2), eines Zulaufteils (3, 4) mit Verstärkungsteil (3) und Abtriebsteil (4), sowie eines Teils (5) auf, wobei die Trennwand T bis zum Boden der Kolonne ausgebildet ist, wobei die Zuführung des Sumpfaustrags von K30 im oberen oder mittleren Drittel, besonders oberen Drittel, des Zulaufteils (3, 4), bezogen auf die Zahl der theoretischen Trennstufen des Zulaufteils, die Abführung von DEG und organischen Produkten mit einem Siedepunkt ≥ 224,8 °C (1,013 bar), bevorzugt > 235 °C (1,013 bar), über Sumpf unterhalb des Teils 5, die Abführung von organischen Produkten mit einem Siedepunkt > 255 °C (1,013 bar) (Hochsieder = HS) über Sumpf unterhalb der Teile 3 und 4, die Abführung von organischen Produkten mit einem Siedepunkt ≤ 224,8 °C (1,013 bar) über Kopf und die Abführung von ADG aus dem mittleren Teil des oberen gemeinsamen Kolonnenbereichs (1) und (2) (Seitenabzug) erfolgt.

Die Trennwandkolonne K60 weist bevorzugt im Bereich von 30 bis 100, besonders im Bereich von 40 bis 90, theoretische Trennstufen auf.
Sie wird bevorzugt bei einem Druck im Bereich von 0,005 bis 1 bar, besonders 0,01 bis 0,7 bar, betrieben.

Der durch die Trennwand (T) unterteilte Teilbereich der Kolonne TK bestehend aus den Teilbereichen 3, 4 und 5, bzw. 2, 3, 4, und 5, oder jeweils Teilen davon ist bevorzugt mit geordneten Packungen, Füllkörpern und/oder Böden bestückt. Die Trennwand in diesen Teilbereichen ist bevorzugt wärmeisolierend ausgeführt.

In einer bevorzugten Ausführungsform werden in der Kolonne K60 über Kopf abgetrennte organische Produkte, wie z.B. N-(2-Aminoethyl)morpholin, 2-(2-Aminoethoxy)-ethylamin, nicht ausgeschleust, sondern in die Umsetzung von DEG mit Ammoniak zurückgeführt.

In Kolonne K60, mit bis zum Boden der Kolonne ausgebilder Trennwand T, über Sumpf abgetrennte Produkte unterhalb des Teils 5 abgetrennte organische Produkte, wie z.B. DEG, werden bevorzugt in die Umsetzung von DEG mit Ammoniak zurückgeführt.

Der Anteil des zurückgeführten Stromes beträgt bevorzugt 80-100 Gew.-%, besonders bevorzugt 95-100 Gew.-%, des im Kolonnenkopf abgetrennten Destillates.

Das erfindungsgemäße Verfahren ist in besonderen Ausführungsformen bei Einsatz einer Trennwandkolonne (TK) vorteilhaft durch einen geringen Wärmebedarf gegenüber der 3-Kolonnenverschaltung (K60 - K80) sowie durch die Reduzierung der Kolonnenanzahl.

Das im erfindungsgemäßen Verfahren eingesetzte Gemisch, enthaltend Morpholin (MO), Monoaminodiglykol (ADG), Ammoniak und Wasser, und ggf. E-MO und ggf. EDA und ggf. organische Produkte mit einem Siedepunkt > 224,8 °C (1,013 bar) wurde in einer Ausführungsform (A) bevorzugt erhalten durch Umsetzung von Diethylenglykol (DEG) mit Ammoniak in Gegenwart eines Katalysators, enthaltend Cu, Ni und Co auf Zirkoniumdioxid als Träger.
Katalysatoren dieses Typs sind in EP-A-963 975, EP-A-1 106 600 und WO-A-03/076386 (alle BASF AG) beschrieben.

In einem besonders bevorzugten Katalysator enthält die katalytisch aktive Masse des Katalysators vor der Behandlung mit Wasserstoff im Bereich von 20 bis 65 Gew.-% Zirkoniumdioxid (ZrO₂), 1 bis 30 Gew.-%, sauerstoffhaltige Verbindungen des Kupfers, berechnet als CuO, 15 bis 50 Gew.-%, sauerstoffhaltige Verbindungen des Nickels, berechnet als NiO, und 15 bis 50 Gew.-% sauerstoffhaltige Verbindungen des Kobalts, berechnet als CoO.

Die für die Umsetzung von Diethylenglykol (DEG) mit Ammoniak bevorzugte Temperatur im Reaktor liegt im Bereich von 170 bis 220 °C. Bevorzugt ist eine isotherme Reaktorfahrweise. Der für die Umsetzung von Diethylenglykol (DEG) mit Ammoniak bevorzugte Druck liegt im Bereich von 100 bis 200 bar.

Bevorzugt wird die Umsetzung von Diethylenglykol (DEG) mit Ammoniak in Gegenwart von Wasserstoff durchgeführt. Der Wasserstoff wird bevorzugt über einen Hochdruckabscheider als Kreisgas in den Reaktor zurückgeführt.
Das Molverhältnis Ammoniak : DEG liegt bevorzugt im Bereich von 4 bis 6.
Der DEG-Umsatz liegt bevorzugt im Bereich von 55 bis 90 %.

Besonders bevorzugt erfolgt die Herstellung des im erfindungsgemäßen Verfahren eingesetzten Gemisches gemäß der älteren deutsche Patentanmeldung Nr. 102005047458.6 vom 30.09.05 und der älteren europäischen (Folge)Patentanmeldung Nr. 06101339.7 vom 06.02.06 (BASF AG), wonach die Umsetzung von DEG mit Ammoniak in Gegenwart eines spezifischen Katalysatorformkörpers erfolgt und die katalytisch aktive Masse des Katalysators vor der Behandlung mit Wasserstoff sauerstoffhaltige Verbindungen des Aluminiums und/oder Zirkoniums, Kupfers, Nickels und Kobalts enthält.

In einer anderen Ausführungsform (B) wurde das im erfindungsgemäßen Verfahren eingesetzte Gemisch, enthaltend Morpholin (MO), Monoaminodiglykol (ADG), Ammoniak und Wasser, und ggf. E-MO und ggf. EDA und ggf. organische Produkte mit einem Siedepunkt > 224,8 °C (1,013 bar), bevorzugt erhalten durch Umsetzung von Diethylenglykol (DEG) mit Ammoniak in Gegenwart eines Katalysators, enthaltend Cu und Ni auf Aluminiumoxid als Träger, wie insbesondere in EP-A-70 397 (BASF AG) beschrieben.
Katalysatoren dieses Typs sind auch in EP-A-514 692 und EP-A-167 872 (beide BASF AG) beschrieben.

In einem hierbei besonders bevorzugten Katalysator enthält die katalytisch aktive Masse des Katalysators vor der Behandlung mit Wasserstoff im Bereich von 25 bis 65 Gew.-% Aluminiumoxid (Al₂O₃), 30 bis 60 Gew.-%, sauerstoffhaltige Verbindungen des Kupfers, berechnet als CuO, und 5 bis 15 Gew.-%, sauerstoffhaltige Verbindungen des Nickels, berechnet als NiO.

Die für die Umsetzung von Diethylenglykol (DEG) mit Ammoniak bevorzugte Temperatur im Reaktor liegt hierbei im Bereich von 190-235 °C. Bevorzugt ist eine isotherme Reaktorfahrweise. Der für die Umsetzung von Diethylenglykol (DEG) mit Ammoniak bevorzugte Druck liegt im Bereich von 20 bis 30 bar.

Bevorzugt wird die Umsetzung von Diethylenglykol (DEG) mit Ammoniak in der Gasphase in Gegenwart von Wasserstoff durchgeführt. Der gasförmige Reaktoraustrag wird bevorzugt in einen Wärmeüberträger geführt, in dem bevorzugt eine partielle Kondensation durchgeführt wird (Kondensation von Roh-Morpholin). Die Gasphase, bestehend aus H₂ und NH₃, wird bevorzugt wieder in den DEG-Verdampfer gefahren und danach in den Reaktor.

Das erfindungsgemäße Verfahren ist insbesondere vorteilhaft zur Herstellung von Morpholin (MO)
mit einer Reinheit von ≥ 99,5 Gew.-%, besonders ≥ 99,6 Gew.-%, z.B. 99,65 bis 99,95 Gew.-%,
einem Gehalt an N-Ethyl-morpholin (E-MO) von ≤ 0,20 Gew.-%, besonders ≤ 0,10 Gew.-%, z.B. 0,01 bis 0,08 Gew.-%,
einem Gehalt an 1,2-Ethylendiamin (EDA) von ≤ 0,30 Gew.-%, besonders ≤ 0,20 Gew.-%, z.B. 0,05 bis 0,15 Gew.-%,
einem Gehalt an 2-Methoxy-ethanol von < 0,50 Gew.-%, besonders < 0,30 Gew.-%, z.B. 0,05 bis 0,25 Gew.-%,
und einem Gehalt an Wasser von ≤ 0,05 Gew.-%, besonders ≤ 0,04 Gew.-%, z.B. 0,01 bis 0,03 Gew.-%.

Ganz besonders ist es vorteilhaft zur Herstellung von Morpholin (MO) mit einer APHA-Farbzahl von ≤ 10, besonders ≤ 8, z.B. 2 bis 7,
und einem Chloridgehalt von ≤ 15 mg/Liter, besonders ≤ 5 mg/Liter, ganz besonders ≤ 1 mg/Liter, z.B. 0,1 bis 0,9 mg/Liter.

Das erfindungsgemäße Verfahren ist weiter insbesondere vorteilhaft zur Herstellung von Monoaminodiglykol (ADG) mit einer Reinheit von ≥ 98,00 Gew.-%, besonders ≥ 98,30 Gew.-%, z.B. 98,50 bis 99,50 Gew.-%,
einem Gehalt an DEG von ≤ 0,40 Gew.-%, besonders ≤ 0,10 Gew.-%, z.B. 0,01 bis 0,08 Gew.-%,
einem Gehalt an Wasser von ≤ 0,20 Gew.-%, besonders ≤ 0,10 Gew.-%, z.B. 0,01 bis 0,08 Gew.-%,
und einer APHA-Farbzahl von ≤ 20, besonders ≤ 15, ganz besonders ≤ 10, z.B. 2 bis 8.

Das erfindungsgemäße Verfahren ist weiter insbesondere vorteilhaft zur Herstellung von N-Ethyl-morpholin (E-MO) mit einer Reinheit von ≥ 98,50 Gew.-%, besonders ≥ 99,00 Gew.-%, z.B. 99,50 bis 99,90 Gew.-%,
einem Gehalt an Wasser von ≤ 0,30 Gew.-%, besonders ≤ 0,20 Gew.-%, z.B. 0,05 bis 0,15 Gew.-%,
und einer APHA-Farbzahl von ≤ 50, besonders ≤ 20, ganz besonders ≤ 10, z.B. 2 bis 8.

APHA-Farbzahlen werden bestimmt gemäß DIN EN 1557.
Der Wassergehalt wird bestimmt gemäß DIN 51777 (K. Fischer).
Der Chloridgehalt wird mittels Ionenchromatographie bestimmt (Detektion von Leitfähigkeit mit chemischer Suppression), nach folgender Methode:

Probenvorbereitung: Ca. 2 g Probe werden in einen Messkolben (10 ml) eingewogen und mit Eluent bis zur Marke aufgefüllt.
Messbedingungen:
lonenchromatographie-System: Metrohm Modulares System (733)
Vorsäule: z.B. DIONEX AG 12; Trennsäule: z.B. DIONEX AS 12
Eluent: z.B. 2,7 mmol Na₂CO₃, 0,28 mmol/l NaHCO₃ in Wasser
Fluss: 1 ml/min; Dosiervolumen: 100 µl
Detektion: Leitfähigkeit nach chemischer Suppression
Suppressor: Metrohm Modul 753
Regenerent: 50 mmol H₂SO₄ in Reinstwasser, (Fluss ca. 0,4 ml/min)
Kalibration: Extern, überprüft durch Aufstock-Versuche
Bestimmungsgrenze: 0,1 mg/kg Chlorid in der Probe.

Im Wertprodukt Morpholin wird der Gehalt an Morpholin, 1,2-Ethylendiamin, N-Ethylmorpholin, 2-Methoxy-ethanol mittels GC bestimmt (GC-Bedingungen: 30 m DB-1; Temperaturprogramm mit 60 °C Anfangstemperatur, 4°C/min. Heizrate, 190 °C Endtemperatur).

Im Wertprodukt ADG wird der Gehalt an ADG und DEG in mittels GC bestimmt (GC-Bedingungen: 30 m DB1, Temperaturprogramm mit 100 °C Anfangstemperatur, 8°C/min Heizrate, 250 °C Endtemperatur).

Zu den Abbildungen:

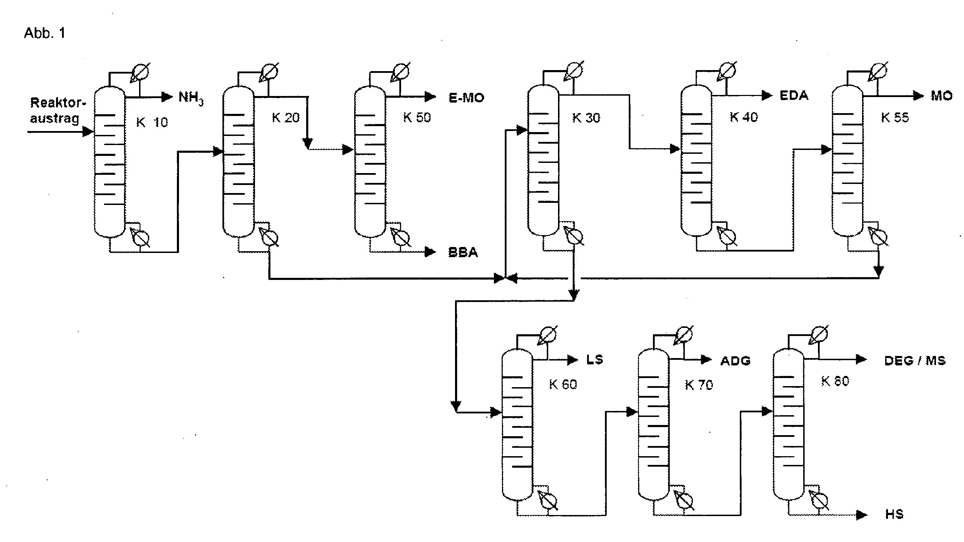

Abbildung 1 zeigt u.a. die erfindungsgemäße Gewinnung von MO und ADG durch eine 9-Kolonnen-Verschaltung.

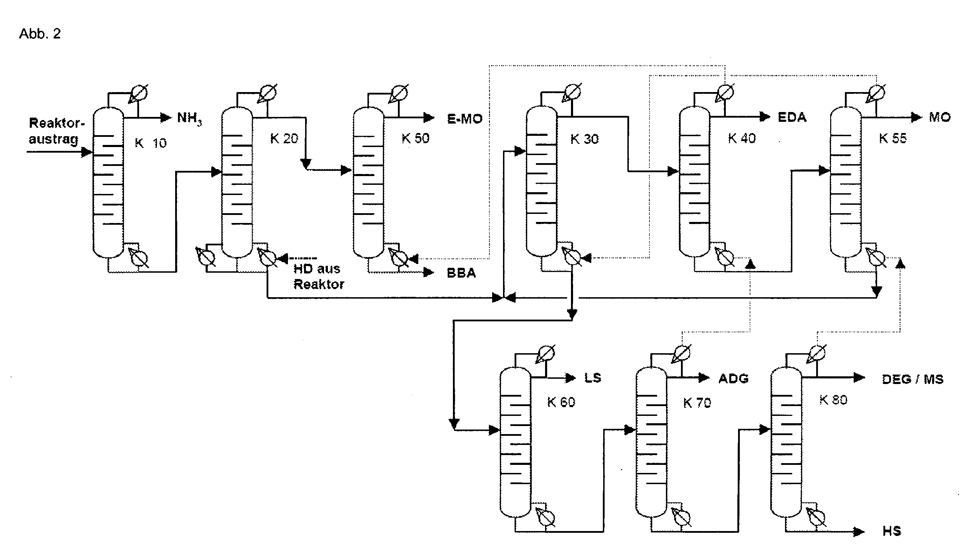

Abbildung 2 zeigt u.a. eine vorteilhafte Ausführungsform der Wärmeintegration, gezeigt durch die gepunkteten Linien.

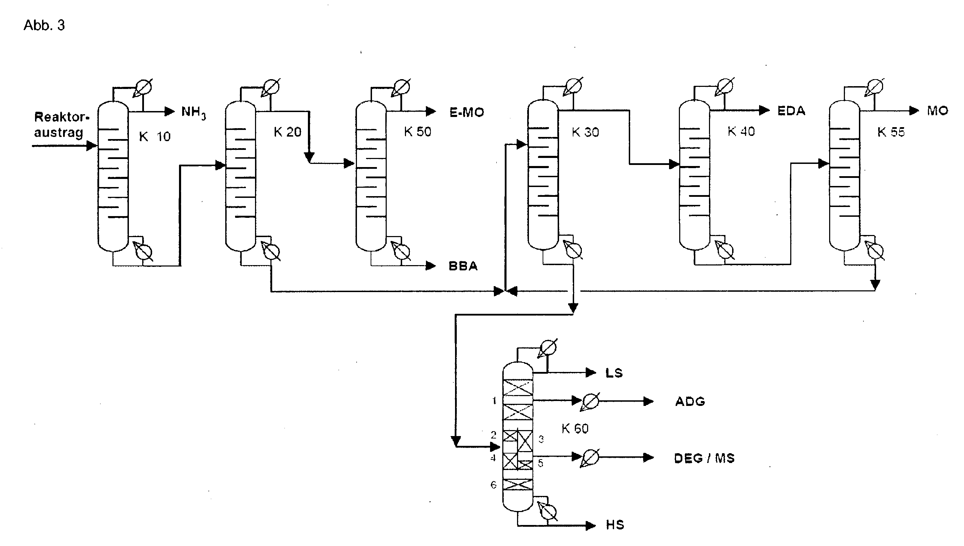

Abbildung 3 zeigt u.a. den Ersatz der Kolonnen K60 - K80 der 9-Kolonnen-Verschaltung durch eine Trennwandkolonne (TK).

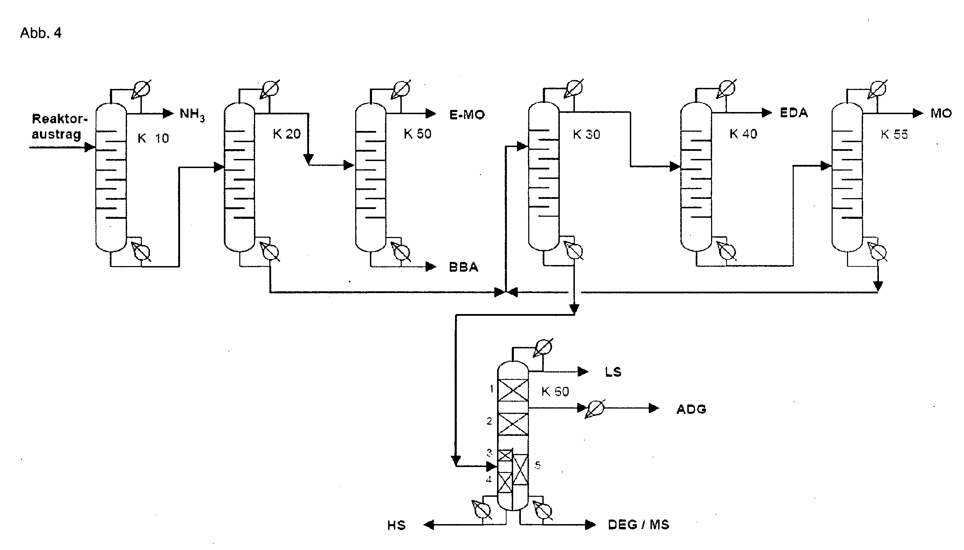

Abbildung 4 zeigt u.a. eine besondere Ausführungsform der Trennwandkolonne K60, in der die Trennwand (T) bis zum Boden der Kolonne ausgebildet ist.
Abbildung 5 zeigt u.a. die 9-Kolonnen-Verschaltung gemäß des Beispiels, inkl. Rückführströmen und Syntheseteil und Wärmeintegration (B1 = Hochdruckabscheider, B2 = Mitteldruckabscheider, C1 = Reaktor, V1 = Verdichter, W1,2,3 und 4 = Wärmetauscher).
Abbildung 6 zeigt u.a. gegenüber Abbildung 5 zusätzlich eine Ausführungsform der Wärmeintegration, gezeigt durch die gepunkteten Linien.

HS = Hochsieder, LS = Leichtsieder, MS = Mittelsieder, BBA = behandlungsbedürftiges Abwasser, HD = Heizdampf.

### Beispiele

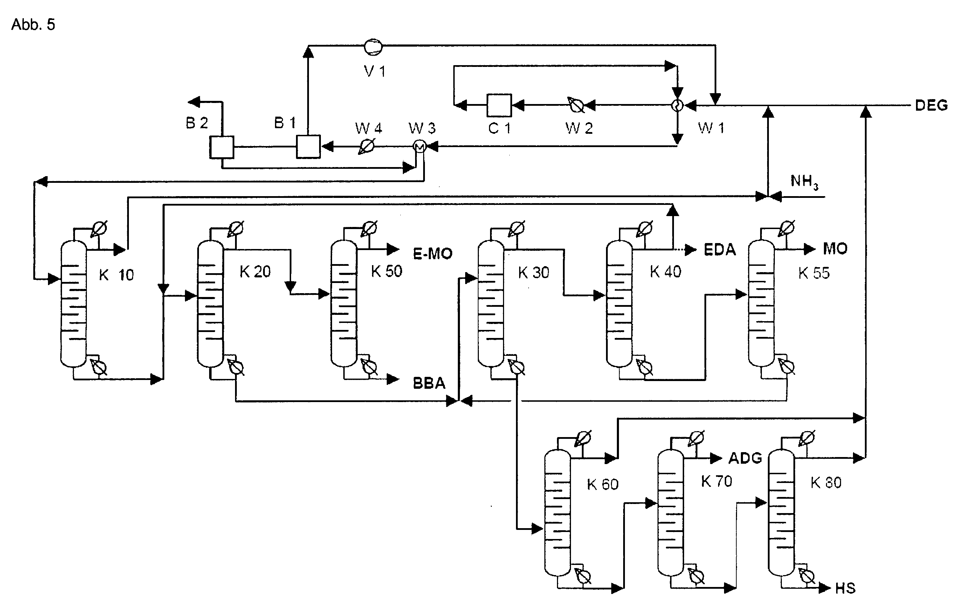

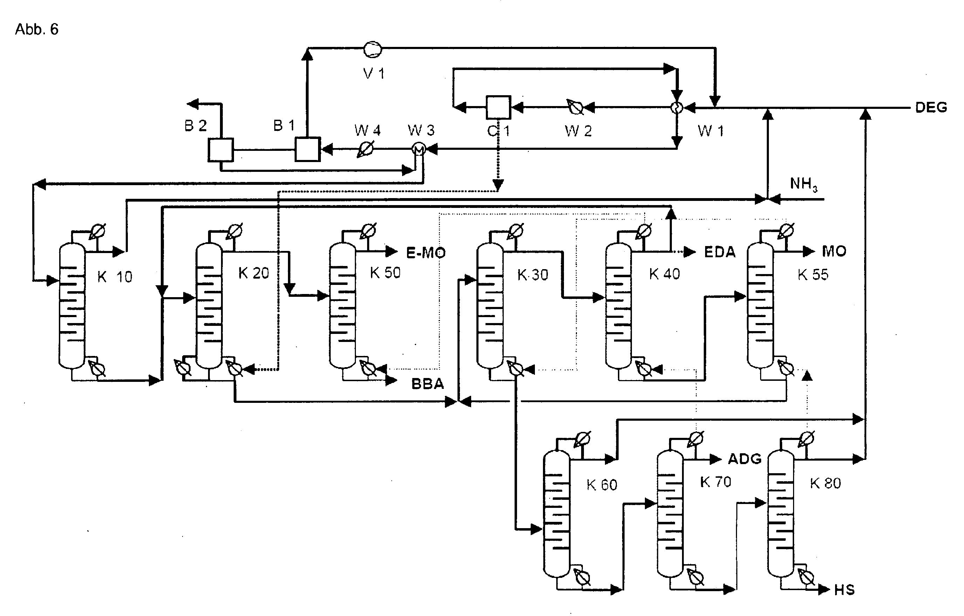

### Beispiel 1 (siehe Abbildungen 5 und 6)

Diglykol (DEG) wird mit dem Kopfprodukt der Kolonne K80 (Hauptkomponenten Diglykol und Morpholyl-ADG) gemischt und kontinuierlich dem Wärmetauscher W 1 zugeführt.
Flüssiges Ammoniak wird mit zurückgeführtem Ammoniak aus der Kolonne K10 gemischt und kontinuierlich dem Wärmetauscher W 1 zugeführt.
Beide Ströme werden vor dem Wärmetauscher W 1 mit dem überwiegend aus Wasserstoff bestehendem Kreisgas vermischt. Das Kreisgas wird von dem am Syntheseausgang gelegenen Hochdruckabscheider B 1 mit dem Verdichter V 1 herangeführt. Vom Wärmetauscher W 1 wird das Gemisch mit einer Temperatur von 145 °C durch einen Aufheizer W 2 auf 195 °C erwärmt und zum Reaktor C 1 gefördert. Am dortigen Festbett-Katalysator erfolgt die Umsetzung des Diglykols zu Aminodiglykol und Morpholin bei einem Druck von 200 bar und Temperaturen bis zu 215 °C. Der Reaktor wird isotherm betrieben. Die Reaktionswärme wird über eine Siedekühlung am Reaktor abgeführt. Dazu wird Wasser bei 14 bar_{abs.} verdampft. Der Dampf wird als Heizdampf an der K20 verwendet. Der Reaktoraustrag wird dann in den Wärmetauschern W 1, W 3 und dem Luftkühler W 4 auf 45 °C abgekühlt. In dem Hochdruckabscheider B 1 erfolgt die Trennung in Gas- und Flüssigphase. Die Gasphase wird - wie oben beschrieben - als Kreisgas zum Wärmetauscher W 1 geführt.

Die flüssige Phase wird aus dem Hochdruckabscheider B 1 in den Mitteldruckabscheider B 2 auf 25 bar entspannt. Das dort frei werdende sogenannte Sprudelgas wird zur NH₃-Rückgewinnung in einen Absorber geleitet. Die zu ergänzende Wasserstoffmenge wird dem Netz entnommen und am Synthesezulauf eingespeist.

Aus dem Mitteldruckabscheider B 2 gelangt das Reaktionsgemisch dann über den Wärmetauscher W 3 in die Kolonne K10.

### Ammoniakabtrennung (K10)

In der Kolonne K10 wird das Ammoniak mit einer Reinheit > 99,9 % bei einem Kopfdruck von 16 bar_{abs.} aus dem Zulauf abdestilliert und auf den Reaktoreingang zurückgeführt. Das Abgas ist an einen Absorber angeschlossen. Der ammoniakfreie Sumpf mit einer Temperatur von 220 °C wird zur Abtrennung von Wasser in die Kolonne K20 entspannt. Die K10 hat 17 theoretische Trennstufen, der Zulauf erfolgt auf Stufe 14.

### Wasserabtrennung (K20)

In der Kolonne K20 wird das Reaktionswasser bei Normaldruck abgetrennt. Das Destillat, das 98 Gew.-% Wasser und 2 Gew.-% Leichtsieder (überwiegend Ethylmorpholin) enthält, wird zur Kolonne K50 geführt. Darüber hinaus wird der Kolonne K20 das Kopfprodukt der LS-Abtrennung in K40 (Hauptkomponenten: 1,2-Ethylendiamin, Morpholin und Wasser) zugeführt. Der weitgehend wasserfreie Sumpf der K20 mit einer Temperatur von 158 °C (Hauptkomponenten: Morpholin, Aminodiglykol, Diglykol und hochsiedender Rückstand) wird zur Kolonne K30 entspannt. Die K20 hat 56 theoretische Trennstufen, der Zulauf erfolgt auf Stufe 25. Die K20 wird zum Teil mit Heizdampf aus der Siedekühlung des Reaktors betrieben. Der zusätzliche Wärmebedarf wird mit Heizdampf aus dem Netz abgedeckt.

### LS/HS-Abtrennung (K30)

In der Kolonne K30 wird der Zulauf, der aus dem Sumpfabzug der Kolonne K20 und dem zurückgeführten Sumpfabzug der Reindestillation K55 besteht, bei einem Kopfdruck von 120 mbar in eine Leichtsiederfraktion (Hauptkomponente: Morpholin) und eine Hochsiederfraktion mit einer Sumpftemperatur von 164 °C (Hauptkomponenten: Aminodiglykol, Diglykol und hochsiedender Rückstand) aufgetrennt. Der Sumpf wird der Kolonne K60 zugeführt. Das Kondensat mit 95 Gew.-% Morpholin, 4,5 Gew.-% 1,2-Ethylendiamin sowie 2-Methoxy-ethanol und Wasser wird der Kolonne K40 zugeführt.
Die K30 hat 14 theoretische Trennstufen, der Zulauf erfolgt auf Stufe 11.

### LS-Abtrennung (K40)

In der Kolonne K40 werden bei einem Kopfdruck von 3,4 bar_{abs}. Leichtsieder (LS) über Kopf abgetrennt. Das Destillat (Hauptkomponenten: 1,2-Ethylendiamin, Morpholin und Wasser) wird zur K20 zurückgeführt bzw. nach Anreicherung des Ethylendiamins diskontinuierlich über einen Behälter ausgeschleust. Der Sumpf der Kolonne K40 mit einer Temperatur von 174 °C (Morpholin mit schwerer siedenden Nebenkomponenten) geht zur K55.
Die K40 hat 38 theoretische Trennstufen, der Zulauf erfolgt auf Stufe 15 Die Wärme aus den Brüden der K40 ist in den Sumpf der K50 integriert.

### Morpholin-Reindestillation (K55)

In der Kolonne K55 wird bei einem Kopfdruck von 4,25 bar_{abs.} Morpholin mit einer Reinheit > 99,6 Gew.% und einem Gehalt an 1,2-Ethylendiamin (EDA) < 0,10 Gew.-% über Kopf abgetrennt. Der Sumpf der Kolonne mit einer Temperatur von 184 °C wird auf den Zulauf der Kolonne K30 zurückgeführt. Die K55 hat 8 theoretische Stufen, der Zulauf erfolgt auf Stufe 5. Die Wärme aus den Brüden der K55 ist in den Sumpf der K30 integriert.

### Ethylmorpholin-Destillation (K50)

In der Kolonne K50 wird bei Normaldruck N-Ethylmorpholin als Azeotrop mit Wasser aus dem Zulauf abgetrennt. Der Sumpf der Kolonne mit einer Temperatur von 103 °C wird ausgeschleust. Die K50 hat 21 theoretische Trennstufen, der Zulauf erfolgt auf Stufe 11.

### LS-Abtrennung (K60)

In der Kolonne K60 werden aus dem Zulauf Leichtsieder bei einem Kopfdruck 160 mbar abgetrennt. Das Destillat der Kolonne (Hauptkomponenten: Aminoethoxyethylamin, Aminoethylmorpholin, Methoxyethylmorpholin sowie geringe Mengen an Aminodiglykol) wird in die Umsetzung von DEG mit Ammoniak zurückgeführt. Der Sumpf der Kolonne mit den Hauptkomponenten Aminodiglykol, Diethylenglykol und einer Sumpftemperatur von 181 °C wird in die nachfolgende K70 geführt. Die K60 hat 18 theoretische Trennstufen, der Zulauf erfolgt auf Stufe 10.

### Aminodiglykol-Destillation (K70)

In der Kolonne K70 wird aus dem Zulauf Aminodiglykol mit einer Reinheit > 98,5 Gew.-% und einem Gehalt an DEG < 0,10 Gew.-% bei einem Kopfdruck von 475 mbar als Destillat gewonnen. Der Sumpf der Kolonne mit einer Sumpftemperatur von 225 °C (64 Gew.-% DEG, 22 Gew.-% Morpholyl-ADG, 5 Gew.% Morpholinodiglykol und 9 Gew.-% Hochsiedern) wird in die K80 geführt. Die K70 hat 33 theoretische Trennstufen, der Zulauf erfolgt auf Stufe 22. Die Wärme aus den Brüden der K70 ist in den Sumpf der K40 integriert.

### HS-Ausschleusung (K80)

In der Kolonne K80 wird am Kopf Diethylenglykol mit einer Reinheit von ∼ 80 % bei einem Kopfdruck von 350 mbar gewonnen. Das Destillat, das neben Diethylenglykol noch Morpholylaminodiglykol enthält wird auf den Eingang des Reaktors zurückgeführt. Die im Sumpf angereicherten Hochsieder (HS) bei einer Sumpftemperatur von ca. 240°C werden aus dem Verfahren ausgeschleust. Die Kolonne hat 28 theoretische Stufen, der Zulauf erfolgt auf Stufe 15. Die Wärme aus den Brüden der K80 ist in den Sumpf der K55 integriert.

Zählweise der Stufen in den Kolonnen ist von unten nach oben.

## Patentansprüche

1. Verfahren zur kontinuierlichen destillativen Auftrennung von Gemischen enthaltend Morpholin (MO), Monoaminodiglykol (ADG), Ammoniak und Wasser, erhalten durch Umsetzung von Diethylenglykol (DEG) mit Ammoniak, **dadurch gekennzeichnet, dass**
in einer ersten Destillationskolonne K10 Ammoniak über Kopf abgetrennt wird, der Sumpfaustrag von K10 einer zweiten Destillationskolonne K20 zugeführt wird, in der Wasser und organische Produkte über Kopf bei einer Kopftemperatur im Bereich von 45 bis 198 °C und einem Druck im Bereich von 0,1 bis 15 bar abgetrennt werden,
der Sumpfaustrag von K20 einer dritten Destillationskolonne K30 zugeführt wird, in der MO und organische Produkte mit einem Siedepunkt < 140 °C (1,013 bar) über Kopf oder in einem Seitenabzug abgetrennt werden und ADG und organische Produkte mit einem Siedepunkt > 190 °C (1,013 bar) über Sumpf abgetrennt werden,
der MO enthaltende Strom, der bei der Kolonne K30 über Kopf oder im Seitenabzug abgetrennt wird, einer vierten Kolonne K40 zugeführt wird, in der organische Produkte mit einem Siedepunkt ≤ 128 °C (1,013 bar) über Kopf und MO und organische Produkte mit einem Siedepunkt ≥ 128 °C (1,013 bar) über Sumpf abgetrennt werden, und
der Sumpfaustrag von K40 einer fünften Destillationskolonne K55 zugeführt wird, in der MO über Kopf und organische Produkte mit einem Siedepunkt ≥ 128 °C (1,013 bar) über Sumpf abgetrennt werden.

2. Verfahren nach dem vorhergehenden Anspruch, **dadurch gekennzeichnet, dass** die in Kolonne K55 über Sumpf abgetrennten Produkte ganz oder teilweise in den Zulauf zur Kolonne K30 zurückgeführt werden.

3. Verfahren nach einem der beiden vorhergehenden Ansprüche, **dadurch gekennzeichnet, dass** die in Kolonne K40 über Kopf abgetrennten Produkte ganz oder teilweise in den Zulauf zur Kolonne K20 zurückgeführt werden.

4. Verfahren nach einem der vorhergehenden Ansprüche, **dadurch gekennzeichnet, dass** die Kolonne K10 im Bereich von 3 bis 30 theoretische Trennstufen aufweist und bei einem Druck im Bereich von 5 bis 30 bar betrieben wird.

5. Verfahren nach einem der vorhergehenden Ansprüche, **dadurch gekennzeichnet, dass** die Kolonne K20 im Bereich von 25 bis 70 theoretische Trennstufen aufweist und bei einem Druck im Bereich von 0,1 bis 10 bar betrieben wird.

6. Verfahren nach einem der vorhergehenden Ansprüche, **dadurch gekennzeichnet, dass** die Kolonne K30 im Bereich von 5 bis 25 theoretische Trennstufen aufweist und bei einem Druck im Bereich von 0,01 bis 5 bar betrieben wird.

7. Verfahren nach einem der vorhergehenden Ansprüche, **dadurch gekennzeichnet, dass** die Kolonne K40 im Bereich von 10 bis 80 theoretischen Trennstufen aufweist und bei einem Druck im Bereich von 0,01 bis 12 bar betrieben wird.

8. Verfahren nach einem der vorhergehenden Ansprüche, **dadurch gekennzeichnet, dass** die Kolonne K55 im Bereich von 1 bis 15 theoretischen Trennstufen aufweist und bei einem Druck im Bereich von 0,01 bis 12 bar betrieben wird.

9. Verfahren nach einem der vorhergehenden Ansprüche, **dadurch gekennzeichnet, dass** sich die Zulaufstelle bei Kolonne K10 im oberen Drittel befindet, bezogen auf die Zahl der theoretischen Trennstufen.

10. Verfahren nach einem der vorhergehenden Ansprüche, **dadurch gekennzeichnet, dass** sich die Zulaufstelle bei Kolonne K20 im mittleren Drittel befindet, bezogen auf die Zahl der theoretischen Trennstufen.

11. Verfahren nach einem der vorhergehenden Ansprüche, **dadurch gekennzeichnet, dass** sich die Zulaufstelle bei Kolonne K30 im oberen Drittel befindet, bezogen auf die Zahl der theoretischen Trennstufen.

12. Verfahren nach einem der vorhergehenden Ansprüche, **dadurch gekennzeichnet, dass** sich die Zulaufstelle bei Kolonne K40 im unteren oder mittleren Drittel befindet, bezogen auf die Zahl der theoretischen Trennstufen.

13. Verfahren nach einem der vorhergehenden Ansprüche, **dadurch gekennzeichnet, dass** sich die Zulaufstelle bei Kolonne K55 im unteren oder mittleren Drittel befindet, bezogen auf die Zahl der theoretischen Trennstufen.

14. Verfahren nach einem der vorhergehenden Ansprüche, **dadurch gekennzeichnet, dass** der wasser- und organische Produkte enthaltende Strom, der bei der Kolonne K20 über Kopf abgetrennt wird, einer Kolonne K50 zugeführt wird, in der wässrige N-Ethyl-morpholin-Lösung (wässrige E-MO-Lösung) über Kopf oder einen flüssigen Seitenabzug und Wasser über Sumpf abgetrennt wird.

15. Verfahren nach dem vorhergehenden Anspruch, **dadurch gekennzeichnet, dass** die Kolonne K50 im Bereich von 10 bis 50 theoretischen Trennstufen aufweist und bei einem Druck im Bereich von 0,1 bis 16 bar betrieben wird.

16. Verfahren nach einem der beiden vorhergehenden Ansprüche, **dadurch gekennzeichnet, dass** sich die Zulaufstelle bei Kolonne K50 im oberen oder mittleren Drittel befindet, bezogen auf die Zahl der theoretischen Trennstufen.

17. Verfahren nach einem der drei vorhergehenden Ansprüche, **dadurch gekennzeichnet, dass** die wässrige N-Ethyl-morpholin-Lösung entwässert und danach die hierbei entstehende organische Phase durch Destillation zum Wertprodukt aufkonzentriert wird.

18. Verfahren nach einem der vorhergehenden Ansprüche, **dadurch gekennzeichnet, dass** der Sumpfaustrag von K30 einer Destillationskolonne K60 zugeführt wird, in der organische Produkte mit einem Siedepunkt ≤ 224,8 °C (1,013 bar) über Kopf und ADG und organische Produkte mit einem Siedepunkt ≥ 224,8 °C (1,013 bar) über Sumpf abgetrennt werden.

19. Verfahren nach dem vorhergehenden Anspruch, **dadurch gekennzeichnet, dass** die Kolonne K60 im Bereich von 5 bis 40 theoretischen Trennstufen aufweist und bei einem Druck im Bereich von 0,005 bis 1 bar betrieben wird.

20. Verfahren nach einem der beiden vorhergehenden Ansprüche, **dadurch gekennzeichnet, dass** sich die Zulaufstelle bei Kolonne K60 im oberen oder mittleren Drittel befindet, bezogen auf die Zahl der theoretischen Trennstufen.

21. Verfahren nach einem der drei vorhergehenden Ansprüche, **dadurch gekennzeichnet, dass** in der Kolonne K60 über Kopf abgetrennte organische Produkte ausgeschleust oder in die Umsetzung von DEG mit Ammoniak zurückgeführt werden.

22. Verfahren nach einem der vier vorhergehenden Ansprüche, **dadurch gekennzeichnet, dass** der ADG enthaltende Strom, der bei der Kolonne K60 im Sumpfabzug abgetrennt wird, einer Kolonne K70 zugeführt wird, in der ADG über Kopf und DEG und organische Produkte mit einem Siedepunkt ≥ 255 °C (1,013 bar) über Sumpf abgetrennt werden.

23. Verfahren nach dem vorhergehenden Anspruch, **dadurch gekennzeichnet, dass** die Kolonne K70 im Bereich von 10 bis 60 theoretische Trennstufen aufweist und bei einem Druck im Bereich von 0,005 bis 1 bar betrieben wird.

24. Verfahren nach einem der beiden vorhergehenden Ansprüche, **dadurch gekennzeichnet, dass** sich die Zulaufstelle bei Kolonne K70 im oberen oder mittleren Drittel befindet, bezogen auf die Zahl der theoretischen Trennstufen.

25. Verfahren nach einem der drei vorhergehenden Ansprüche, **dadurch gekennzeichnet, dass** der DEG enthaltende Strom, der bei der Kolonne K70 im Sumpfabzug abgetrennt wird, einer Kolonne K80 zugeführt wird, in der DEG über Kopf und organische Produkte mit einem Siedepunkt ≥ 255 °C (1,013 bar) über Sumpf abgetrennt werden.

26. Verfahren nach dem vorhergehenden Anspruch, **dadurch gekennzeichnet, dass** die Kolonne K80 im Bereich von 10 bis 60 theoretische Trennstufen aufweist und bei einem Druck im Bereich von 0,005 bis 1 bar betrieben wird.

27. Verfahren nach einem der beiden vorhergehenden Ansprüche, **dadurch gekennzeichnet, dass** sich die Zulaufstelle bei Kolonne K80 im oberen oder mittleren Drittel befindet, bezogen auf die Zahl der theoretischen Trennstufen.

28. Verfahren nach einem der drei vorhergehenden Ansprüche, **dadurch gekennzeichnet, dass** in Kolonne K80 über Kopf abgetrennte Produkte in die Umsetzung von DEG mit Ammoniak zurückgeführt werden.

29. Verfahren nach einem der Ansprüche 1 bis 17, **dadurch gekennzeichnet, dass** der Sumpfaustrag von K30 einer Destillationskolonne K60 zugeführt wird, wobei es sich bei der Kolonne K60 um eine Trennwandkolonne (TK) handelt, die eine Trennwand (T) in Kolonnenlängsrichtung unter Ausbildung eines oberen gemeinsamen Kolonnenbereichs (1), eines unteren gemeinsamen Kolonnenbereichs (6), eines Zulaufteils (2, 4) mit Verstärkungsteil (2) und Abtriebsteil (4), sowie eines Entnahmeteils (3, 5) mit Verstärkungsteil (3) und Abtriebsteil (5) aufweist, wobei die Zuführung des Sumpfaustrags von K30 im oberen oder mittleren Drittel des Zulaufteils (2, 4), bezogen auf die Zahl der theoretischen Trennstufen des Zulaufteils, die Abführung von organischen Produkten mit einem Siedepunkt > 255 °C (1,013 bar) über Sumpf, die Abführung von organischen Produkten mit einem Siedepunkt ≤ 224,8 °C (1,013 bar) über Kopf, die Abführung von ADG aus dem Kolonnenbereich 1 und optional die Abführung von dampfförmigen organischen Produkten mit einem Siedepunkt ≥ 224,8 °C (1,013 bar), wie DEG, aus dem oberen oder mittleren Drittel des Entnahmeteils (3, 5) (Seitenabzug), bezogen auf die Zahl der theoretischen Trennstufen des Entnahmeteils, erfolgt.

30. Verfahren nach dem vorhergehenden Anspruch, **dadurch gekennzeichnet, dass** über den dampfförmigen Seitenabzug abgetrennte Produkte in die Umsetzung von DEG mit Ammoniak zurückgeführt werden.

31. Verfahren nach einem der Ansprüche 1 bis 17, **dadurch gekennzeichnet, dass** der Sumpfaustrag von K30 einer Destillationskolonne K60 zugeführt wird, wobei es sich bei der Kolonne K60 um eine Trennwandkolonne (TK) handelt, die eine Trennwand (T) in Kolonnenlängsrichtung unter Ausbildung eines oberen gemeinsamen Kolonnenbereichs (1) und (2), eines Zulaufteils (3, 4) mit Verstärkungsteil (3) und Abtriebsteil (4), sowie eines Teils (5) aufweist, wobei die Trennwand T bis zum Boden der Kolonne ausgebildet ist, wobei die Zuführung des Sumpfaustrags von K30 im oberen oder mittleren Drittel des Zulaufteils (3, 4), bezogen auf die Zahl der theoretischen Trennstufen des Zulaufteils, die Abführung von DEG und organischen Produkten mit einem Siedepunkt ≥ 224,8 °C (1,013 bar) über Sumpf unterhalb des Teils 5, die Abführung von organischen Produkten mit einem Siedepunkt > 255 °C (1,013 bar) über Sumpf unterhalb der Teile 3 und 4, die Abführung von organischen Produkten mit einem Siedepunkt ≤ 224,8 °C (1,013 bar) über Kopf und die Abführung von ADG aus dem mittleren Teil des oberen gemeinsamen Kolonnenbereichs (1) und (2) (Seitenabzug) erfolgt.

32. Verfahren nach dem vorhergehenden Anspruch, **dadurch gekennzeichnet, dass** über Sumpf abgetrennte Produkte unterhalb des Teils 5 in die Umsetzung von DEG mit Ammoniak zurückgeführt werden.

33. Verfahren nach einem der vier vorhergehenden Ansprüche, **dadurch gekennzeichnet, dass** die Trennwandkolonne K60 im Bereich von 30 bis 100 theoretischen Trennstufen aufweist und bei einem Druck im Bereich von 0,005 bis 1 bar betrieben wird.

34. Verfahren nach einem der fünf vorhergehenden Ansprüche, **dadurch gekennzeichnet, dass** der durch die Trennwand (T) unterteilte Teilbereich der Kolonne (TK) bestehend aus den Teilbereichen 3, 4 und 5, bzw. 2, 3, 4, und 5, oder jeweils Teilen davon mit geordneten Packungen, Füllkörpern und/oder Böden bestückt ist und die Trennwand in diesen Teilbereichen wärmeisolierend ausgeführt ist.

35. Verfahren nach einem der sechs vorhergehenden Ansprüche, **dadurch gekennzeichnet, dass** über Kopf abgetrennte organische Produkte in die Umsetzung von DEG mit Ammoniak zurückgeführt werden.

36. Verfahren nach einem der vorhergehenden Ansprüche zur Auftrennung von Gemischen enthaltend Morpholin (MO), Monoaminodiglykol (ADG), Ammoniak, Wasser, N-Ethyl-morpholin (E-MO), 1,2-Ethylendiamin (EDA) und organische Produkte mit einem Siedepunkt > 224,8 °C (1,013 bar).

37. Verfahren nach einem der vorhergehenden Ansprüche, **dadurch gekennzeichnet, dass** das Gemisch, enthaltend Morpholin (MO), Monoaminodiglykol (ADG), Ammoniak und Wasser, erhalten wurde durch Umsetzung von Diethylenglykol (DEG) mit Ammoniak in Gegenwart eines Heterogenkatalysators, enthaltend Cu, Ni und Co auf Zirkoniumdioxid als Träger oder in Gegenwart eines Heterogenkatalysators, enthaltend Cu und Ni auf Aluminiumoxid als Träger.

38. Verfahren nach einem der vorhergehenden Ansprüche, **dadurch gekennzeichnet, dass** anfallende Wärme der Brüden und/oder anfallende Reaktionswärme aus der Synthese des zur destillativen Auftrennung eingesetzten Gemisches durch Integration in den Verdampfern der Kolonnen K20, K30, K40, K50, K55 und/oder K60 genutzt wird.

39. Verfahren nach einem der vorhergehenden Ansprüche zur Herstellung von Morpholin (MO) mit einer Reinheit von ≥ 99,5 Gew.-%, einem Gehalt an N-Ethylmorpholin (E-MO) von ≤ 0,20 Gew.-%, einem Gehalt an 1,2-Ethylendiamin (EDA) von ≤ 0,30 Gew.-%, einem Gehalt an 2-Methoxy-ethanol von < 0,50 Gew.-% und einem Gehalt an Wasser von ≤ 0,05 Gew.-%.

40. Verfahren nach einem der vorhergehenden Ansprüche zur Herstellung von Morpholin (MO) mit einer APHA-Farbzahl von ≤ 10 und einem Chloridgehalt von ≤ 15 mg/Liter.

41. Verfahren nach einem der Ansprüche 22 bis 40 zur Herstellung von Monoaminodiglykol (ADG) mit einer Reinheit von ≥ 98,00 Gew.-%, einem Gehalt an DEG von ≤ 0,40 Gew.-%, einem Gehalt an Wasser von ≤ 0,20 Gew.-% und einer APHA-Farbzahl von ≤ 20.

42. Verfahren nach einem der Ansprüche 17 bis 41 zur Herstellung von N-Ethylmorpholin (E-MO) mit einer Reinheit von ≥ 98,50 Gew.-%, einem Gehalt an Wasser von ≤ 0,30 Gew.-% und einer APHA-Farbzahl von ≤ 50.

## Claims

1. A process for the continuous fractional distillation of mixtures comprising morpholine (MO), monoaminodiglycol (ADG), ammonia and water obtained by reaction of diethylene glycol (DEG) with ammonia, which comprises
separating off ammonia at the top of a first distillation column K10,
feeding the bottoms from K10 to a second distillation column K20 in which water and organic products are separated off at the top at a temperature at the top in the range from 45 to 198°C and a pressure in the range from 0.1 to 15 bar,
feeding the bottoms from K20 to a third distillation column K30 in which MO and organic products having a boiling point of < 140°C (1.013 bar) are separated off at the top or at a side offtake and ADG and organic products having a boiling point of > 190°C (1.013 bar) are separated off at the bottom,
feeding the MO-comprising stream which is separated off at the top or at a side offtake of the column K30 to a fourth column K40 in which organic products having a boiling point of ≤ 128°C (1.013 bar) are separated off at the top and MO and organic products having a boiling point of ≥ 128°C (1.013 bar) are separated off at the bottom and
feeding the bottoms from K40 to a fifth distillation column K55 in which MO is separated off at the top and organic products having a boiling point of ≥ 128°C (1.013 bar) are separated off at the bottom.

2. The process according to the preceding claim, wherein the products separated off at the bottom of column K55 are recirculated in their entirety or in part to the feed to the column K30.

3. The process according to either of the two preceding claims, wherein the products separated off at the top of column K40 are recirculated in their entirety or in part to the feed to the column K20.

4. The process according to any of the preceding claims, wherein the column K10 has from 3 to 30 theoretical plates and is operated at a pressure in the range from 5 to 30 bar.

5. The process according to any of the preceding claims, wherein the column K20 has from 25 to 70 theoretical plates and is operated at a pressure in the range from 0.1 to 10 bar.

6. The process according to any of the preceding claims, wherein the column K30 has from 5 to 25 theoretical plates and is operated at a pressure in the range from 0.01 to 5 bar.

7. The process according to any of the preceding claims, wherein the column K40 has from 10 to 80 theoretical plates and is operated at a pressure in the range from 0.01 to 12 bar.

8. The process according to any of the preceding claims, wherein the column K55 has from 1 to 15 theoretical plates and is operated at a pressure in the range from 0.01 to 12 bar.

9. The process according to any of the preceding claims, wherein the feed point of column K10 is located in the upper third, based on the number of theoretical plates.

10. The process according to any of the preceding claims, wherein the feed point of column K20 is located in the middle third, based on the number of theoretical plates.

11. The process according to any of the preceding claims, wherein the feed point of column K30 is located in the upper third, based on the number of theoretical plates.

12. The process according to any of the preceding claims, wherein the feed point of column K40 is located in the lower or middle third, based on the number of theoretical plates.

13. The process according to any of the preceding claims, wherein the feed point of column K55 is located in the lower or middle third, based on the number of theoretical plates.

14. The process according to any of the preceding claims, wherein the stream comprising water and organic products which is separated off at the top of the column K20 is fed to a column K50 in which aqueous N-ethylmorpholine solution (aqueous E-MO solution) is separated off at the top or at a side offtake for liquid and water is separated off at the bottom.

15. The process according to the preceding claim, wherein the column K50 has from 10 to 50 theoretical plates and is operated at a pressure in the range from 0.1 to 16 bar.

16. The process according to either of the two preceding claims, wherein the feed point of column K50 is located in the upper or middle third, based on the number of theoretical plates.

17. The process according to any of the three preceding claims, wherein the aqueous N-ethylmorpholine solution is dewatered and the organic phase formed here is then concentrated by distillation to give the desired product.

18. The process according to any of the preceding claims, wherein the bottoms from K30 are fed to a distillation column K60 in which organic products having a boiling point of ≤ 224.8°C (1.013 bar) are separated off at the top and ADG and organic products having a boiling point of ≥ 224.8°C (1.013 bar) are separated off at the bottom.

19. The process according to the preceding claim, wherein the column K60 has from 5 to 40 theoretical plates and is operated at a pressure in the range from 0.005 to 1 bar.

20. The process according to either of the two preceding claims, wherein the feed point of column K60 is located in the upper or middle third, based on the number of theoretical plates.

21. The process according to any of the three preceding claims, wherein organic products separated off at the top of the column K60 are discharged or recirculated to the reaction of DEG with ammonia.

22. The process according to any of the four preceding claims, wherein the ADG-comprising stream separated off at the bottom offtake of the column K60 is fed to a column K70 in which ADG is separated off at the top and DEG and organic products having a boiling point of ≥ 255°C (1.013 bar) are separated off at the bottom.

23. The process according to the preceding claim, wherein the column K70 has from 10 to 60 theoretical plates and is operated at a pressure in the range from 0.005 to 1 bar.

24. The process according to either of the two preceding claims, wherein the feed point of column K70 is located in the upper or middle third, based on the number of theoretical plates.

25. The process according to any of the three preceding claims, wherein the DEG-comprising stream separated off at the bottom offtake of the column K70 is fed to a column K80 in which DEG is separated off at the top and organic products having a boiling point of ≥ 255°C (1.013 bar) are separated off at the bottom.

26. The process according to the preceding claim, wherein the column K80 has from 10 to 60 theoretical plates and is operated at a pressure in the range from 0.005 to 1 bar.

27. The process according to either of the two preceding claims, 1 wherein the feed point of the column K80 is located in the upper or middle third, based on the number of theoretical plates.

28. The process according to any of the three preceding claims, wherein products separated off at the top of column K80 are recirculated to the reaction of DEG with ammonia.

29. The process according to any of claims 1 to 17, wherein the bottoms from K30 are fed to a distillation column K60 which is a dividing wall column (DWC) which has a dividing wall (DW) in the longitudinal direction of the column to form an upper combined column region (1), a lower combined column region (6), an inflow part (2, 4) having an enrichment section (2) and a stripping section (4), and also an offtake part (3, 5) having an enrichment section (3) and a stripping section (5), with the bottoms from K30 being fed in in the upper or middle third of the inflow part (2, 4), based on the number of theoretical plates of the inflow part, organic products having a boiling point of > 255°C (1.013 bar) being discharged at the bottom, organic products having a boiling point of ≤ 224.8°C (1.013 bar) being discharged at the top, ADG being discharged from the column region 1 and optionally gaseous organic products having a boiling point of ≥ 224.8°C (1.013 bar), e.g. DEG, being discharged from the upper or middle third of the offtake part (3, 5) (side offtake), based on the number of theoretical plates of the offtake part.

30. The process according to the preceding claim, wherein products separated off in gaseous form at the side offtake are recirculated to the reaction of DEG with ammonia.

31. The process according to any of claims 1 to 17, wherein the bottoms from K30 are fed to a distillation column K60 which is a dividing wall column (DWC) which has a dividing wall (DW) in the longitudinal direction of the column to form an upper combined column region (1) and (2), an inflow part (3, 4) having an enrichment section (3) and a stripping section (4), and also a part (5), with the dividing wall DW extending to the bottom of the column and the bottoms from K30 being fed in in the upper or middle third of the inflow part (3, 4), based on the number of theoretical plates of the inflow part, DEG and organic products having a boiling point of ≥ 224.8°C (1.013 bar) being discharged at the bottom below the part 5, organic products having a boiling point of > 255°C (1.013 bar) being discharged at the bottom below the parts 3 and 4, organic products having a boiling point of ≤ 224.8°C (1.013 bar) being discharged at the top and ADG being discharged from the middle part of the upper combined column region (1) and (2) (side offtake).

32. The process according to the preceding claim, wherein products separated off at the bottom below the part 5 are recirculated to the reaction of DEG with ammonia.

33. The process according to any of the four preceding claims, wherein the dividing wall column K60 has from 30 to 100 theoretical plates and is operated at a pressure in the range from 0.005 to 1 bar.

34. The process according to any of the five preceding claims, wherein the subregion of the column (DWC) divided by the dividing wall (DW) and comprising the subregions 3, 4 and 5 or 2, 3, 4 and 5 or in each case parts thereof is provided with ordered packing, random packing elements and/or trays and the dividing wall in these subregions is thermally insulating.

35. The process according to any of the six preceding claims, wherein organic products separated off at the top are recirculated to the reaction of DEG with ammonia.

36. The process according to any of the preceding claims for the fractionation of mixtures comprising morpholine (MO), monoaminodiglycol (ADG), ammonia, water, N-ethylmorpholine (E-MO), 1,2-ethylenediamine (EDA) and organic products having a boiling point of > 224.8°C (1.013 bar).

37. The process according to any of the preceding claims, wherein the mixture comprising morpholine (MO), monoaminodiglycol (ADG), ammonia and water has been obtained by reaction of diethylene glycol (DEG) with ammonia in the presence of a heterogeneous catalyst comprising Cu, Ni and Co on zirconium dioxide as support or in the presence of a heterogeneous catalyst comprising Cu and Ni on aluminum oxide as support.

38. The process according to any of the preceding claims, wherein heat obtained in the vapor and/or heat of reaction obtained from the synthesis of the mixture used for the fractional distillation is utilized by integration into the vaporizers of the columns K20, K30, K40, K50, K55 and/or K60.

39. The process according to any of the preceding claims for preparing morpholine (MO) having a purity of ≥ 99.5% by weight, an N-ethylmorpholine (E-MO) content of ≤ 0.20% by weight, a 1,2-ethylenediamine (EDA) content of ≤ 0.30% by weight, a 2-methoxyethanol content of < 0.50% by weight and a water content of ≤ 0.05% by weight.

40. The process according to any of the preceding claims for preparing morpholine (MO) having an APHA color number of ≤ 10 and a chloride content of ≤ 15 mg/liter.

41. The process according to any of claims 22 to 40 for preparing monoaminodiglycol (ADG) having a purity of ≥ 98.00% by weight, a DEG content of ≤ 0.40% by weight, a water content of ≤ 0.20% by weight and an APHA color number of ≤ 20.

42. The process according to any of claims 17 to 41 for preparing N-ethylmorpholine (E-MO) having a purity of ≥ 98.50% by weight, a water content of ≤ 0.30% by weight and an APHA color number of ≤ 50.

## Revendications

1. Procédé pour la séparation continue par distillation de mélanges contenant de la morpholine (MO), du monoaminodiglycol (ADG), de l'ammoniac et de l'eau, obtenus par transformation de diéthylèneglycol (DEG) avec de l'ammoniac, **caractérisé en ce que**
- dans une première colonne de distillation K10, on sépare de l'ammoniac via la tête, on alimente le produit évacué du fond de K10 dans une deuxième colonne de distillation K20 dans laquelle on sépare l'eau et les produits organiques via la tête à une température de la tête dans la plage de 45 à 198°C et à une pression dans la plage de 0,1 à 15 bars,
- on alimente le produit évacué du fond de K20 dans une troisième colonne de distillation K30, dans laquelle on sépare la MO et les produits organiques présentant un point d'ébullition < 140°C (1,013 bar) via la tête ou dans un soutirage latéral et l'ADG et les produits organiques présentant un point d'ébullition > 190°C (1,013 bar) via le fond de colonne,
- on alimente le flux contenant la MO, qui est séparé au niveau de la colonne K30 via la tête ou dans l'évacuation latérale, dans une quatrième colonne K40 dans laquelle on sépare les produits organiques présentant un point d'ébullition ≤ 128°C (1,013 bar) via la tête et la MO et les produits organiques présentant un point d'ébullition ≥ 128°C (1,013 bar) via le fond de colonne,
- on alimente le produit évacué du fond de K40 dans une cinquième colonne de distillation K55, dans laquelle on sépare la MO via la tête et les produits organiques présentant un point d'ébullition ≥ 128°C (1,013 bar) via le fond de colonne.

2. Procédé selon la revendication précédente, **caractérisé en ce que** les produits séparés dans la colonne K55 via le fond sont recyclés totalement ou partiellement dans l'alimentation vers la colonne K30.

3. Procédé selon l'une des deux revendications précédentes, **caractérisé en ce que** les produits séparés dans la colonne K40 via la tête sont recyclés totalement ou partiellement dans l'alimentation vers la colonne K20.

4. Procédé selon l'une quelconque des revendications précédentes, **caractérisé en ce que** la colonne K10 présente 3 à 30 plateaux théoriques de séparation et est exploitée à une pression de 5 à 30 bars.

5. Procédé selon l'une quelconque des revendications précédentes, **caractérisé en ce que** la colonne K20 présente 25 à 70 plateaux théoriques de séparation et est exploitée à une pression de 0,1 à 10 bars.

6. Procédé selon l'une quelconque des revendications précédentes, **caractérisé en ce que** la colonne K30 présente 5 à 25 plateaux théoriques de séparation et est exploitée à une pression de 0,01 à 5 bars.

7. Procédé selon l'une quelconque des revendications précédentes, **caractérisé en ce que** la colonne K40 présente 10 à 80 plateaux théoriques de séparation et est exploitée à une pression de 0,01 à 12 bars.

8. Procédé selon l'une quelconque des revendications précédentes, **caractérisé en ce que** la colonne K55 présente 1 à 15 plateaux théoriques de séparation et est exploitée à une pression de 0,01 à 12 bars.

9. Procédé selon l'une quelconque des revendications précédentes, **caractérisé en ce que** le site d'alimentation de la colonne K10 se trouve dans le tiers supérieur par rapport au nombre de plateaux théoriques de séparation.

10. Procédé selon l'une quelconque des revendications précédentes, **caractérisé en ce que** le site d'alimentation de la colonne K20 se trouve dans le tiers central par rapport au nombre de plateaux théoriques de séparation.

11. Procédé selon l'une quelconque des revendications précédentes, **caractérisé en ce que** le site d'alimentation de la colonne K30 se trouve dans le tiers supérieur par rapport au nombre de plateaux théoriques de séparation.

12. Procédé selon l'une quelconque des revendications précédentes, **caractérisé en ce que** le site d'alimentation de la colonne K40 se trouve dans le tiers inférieur ou central par rapport au nombre de plateaux théoriques de séparation.

13. Procédé selon l'une quelconque des revendications précédentes, **caractérisé en ce que** le site d'alimentation de la colonne K55 se trouve dans le tiers inférieur ou central par rapport au nombre de plateaux théoriques de séparation.

14. Procédé selon l'une quelconque des revendications précédentes, **caractérisé en ce que** le flux contenant les produits aqueux et organiques qui est séparé dans la colonne K20 via la tête, est alimenté dans une colonne K50 dans laquelle une solution aqueuse de N-éthylmorpholine (solution aqueuse E-MO) est séparée via la tête ou un soutirage latéral liquide et l'eau est séparée via le fond.

15. Procédé selon la revendication précédente, **caractérisé en ce que** la colonne K50 présente 10 à 50 plateaux théoriques de séparation et est exploitée à une pression de 0,1 à 16 bars.

16. Procédé selon l'une des deux revendications précédentes, **caractérisé en ce que** le site d'alimentation de la colonne K50 se trouve dans le tiers supérieur ou central par rapport au nombre de plateaux théoriques de séparation.

17. Procédé selon l'une quelconque des trois revendications précédentes, **caractérisé en ce que** la solution aqueuse de N-éthylmorpholine est déshydratée et ensuite, la phase organique ainsi formée est concentrée en produit de valeur par distillation.

18. Procédé selon l'une quelconque des revendications précédentes, **caractérisé en ce qu'**on alimente le produit évacué du fond de K30 dans une colonne de distillation K60, dans laquelle on sépare les produits organiques présentant un point d'ébullition ≤ 224,8°C (2,013 bar) via la tête et l'ADG et les produits organiques présentant un point d'ébullition ≥ 224,8°C (1,013 bar) via le fond.

19. Procédé selon la revendication précédente, **caractérisé en ce que** la colonne K60 présente 5 à 40 plateaux théoriques de séparation et est exploitée à une pression de 0,005 à 1 bar.

20. Procédé selon l'une des deux revendications précédentes, **caractérisé en ce que** le site d'alimentation de la colonne K60 se trouve dans le tiers supérieur ou central par rapport au nombre de plateaux théoriques de séparation.

21. Procédé selon l'une quelconque des trois revendications précédentes, **caractérisé en ce que** les produits organiques séparés dans la colonne K60 via la tête sont évacués ou recyclés dans la transformation de DEG avec de l'ammoniac.

22. Procédé selon l'une quelconque des quatre revendications précédentes, **caractérisé en ce qu'**on alimente le flux contenant l'ADG, qui est séparé au niveau de la colonne K60 dans le produit évacué du fond, dans une colonne K70 dans laquelle on sépare l'ADG via la tête et le DEG et les produits organiques présentant un point d'ébullition ≥ 255°C (1,013 bar) via le fond.

23. Procédé selon la revendication précédente, **caractérisé en ce que** la colonne K70 présente 10 à 60 plateaux théoriques de séparation et est exploitée à une pression de 0,005 à 1 bar.

24. Procédé selon l'une quelconque des deux revendications précédentes, **caractérisé en ce que** le site d'alimentation de la colonne K70 se trouve dans le tiers supérieur ou central par rapport au nombre de plateaux théoriques de séparation.

25. Procédé selon l'une quelconque des trois revendications précédentes, **caractérisé en ce qu'**on alimente le flux contenant le DEG, qui est séparé au niveau de la colonne K70 dans le produit évacué du fond, dans une colonne K80 dans laquelle on sépare le DEG via la tête et les produits organiques présentant un point d'ébullition ≥ 255°C (1,013 bar) via le fond.

26. Procédé selon la revendication précédente, **caractérisé en ce que** la colonne K80 présente 10 à 60 plateaux théoriques de séparation et est exploitée à une pression de 0,005 à 1 bar.

27. Procédé selon l'une quelconque des deux revendications précédentes, **caractérisé en ce que** le site d'alimentation de la colonne K80 se trouve dans le tiers supérieur ou central par rapport au nombre de plateaux théoriques de séparation.

28. Procédé selon l'une quelconque des trois revendications précédentes, **caractérisé en ce que** les produits séparés dans la colonne K80 via la tête sont recyclés dans la transformation de DEG avec de l'ammoniac.

29. Procédé selon l'une quelconque des revendications 1 à 17, **caractérisé en ce que** le produit évacué du fond de K30 est alimenté dans une colonne de distillation K60, où il s'agit, pour la colonne K60, d'une colonne à paroi de séparation (TK) qui présente une paroi de séparation (T) dans le sens longitudinal de la colonne avec formation d'une zone de colonne commune supérieure (1), d'une zone de colonne commune inférieure (6), d'une zone d'alimentation (2, 4) avec une zone de rectification (2) et une zone de dérivation (4), ainsi que d'une zone de prélèvement (3, 5) avec une zone de rectification (3) et une zone de dérivation (5), l'alimentation du produit évacué du fond de K30 étant réalisée dans le tiers supérieur ou central de la zone d'alimentation (2, 4), par rapport au nombre de plateaux théoriques de séparation de la zone d'alimentation, l'évacuation des produits organiques présentant un point d'ébullition > 255°C (1,013 bar) étant réalisée via le fond, l'évacuation des produits organiques présentant un point d'ébullition ≤ 224,8°C (1,013 bar) étant réalisée via la tête, l'évacuation d'ADG de la zone de colonne 1 et éventuellement l'évacuation des produits organiques sous forme de vapeur présentant un point d'ébullition ≥ 224,8°C (1,013 bar), tels que le DEG, étant réalisée du tiers supérieur ou central de la zone de prélèvement (3, 5) (soutirage latéral), par rapport au nombre de plateaux théoriques de séparation de la zone de prélèvement.

30. Procédé selon la revendication précédente, **caractérisé en ce que** les produits séparés via le soutirage latéral sous forme de vapeur sont recyclés dans la transformation de DEG avec de l'ammoniac.

31. Procédé selon l'une quelconque des revendications 1 à 17, **caractérisé en ce que** le produit évacué du fond de K30 est alimenté dans une colonne de distillation K60, où il s'agit, pour la colonne K60, d'une colonne à paroi de séparation (TK) qui présente une paroi de séparation (T) dans le sens longitudinal de la colonne avec formation d'une zone de colonne commune supérieure (1) et (2), d'une zone d'alimentation (3, 4) avec une zone de rectification (3) et une zone de dérivation (4), ainsi que d'une zone (5), où la paroi de séparation T est réalisée jusqu'au fond de la colonne, l'alimentation du produit évacué du fond de K30 étant réalisée dans le tiers supérieur ou central de la zone d'alimentation (3, 4), par rapport au nombre de plateaux théoriques de séparation de la zone d'alimentation, l'évacuation de DEG et des produits organiques présentant un point d'ébullition ≥ 224,8°C (1,013 bar) étant réalisée via le fond sous la zone 5, l'évacuation des produits organiques présentant un point d'ébullition > 225°C (1,013 bar) étant réalisée via le fond sous les zones 3 et 4, l'évacuation des produits organiques présentant un point d'ébullition ≤ 224,8 °C (1,013 bar) étant réalisée via la tête et l'évacuation de l'ADG étant réalisée via la zone centrale de la zone de colonne commune supérieure (1) et (2) (soutirage latéral).

32. Procédé selon la revendication précédente, **caractérisé en ce que** les produits séparés via le fond au-dessous de la zone 5 sont recyclés dans la transformation de DEG avec de l'ammoniac.

33. Procédé selon l'une quelconque des quatre revendications précédentes, **caractérisé en ce que** la colonne à paroi de séparation K60 présente 30 à 100 plateaux théoriques de séparation et est exploitée à une pression de 0,005 à 1 bar.

34. Procédé selon l'une quelconque des cinq revendications précédentes, **caractérisé en ce que** la zone partielle divisée par la paroi de séparation (T) de la colonne (TK) constituée par les zones partielles 3, 4 et 5, ou, selon le cas, 2, 3, 4, et 5, ou à chaque fois des parties de celle-ci est équipée de garnissages structurés, de corps de remplissage et/ou de plateaux et la paroi de séparation est réalisée dans ces zones partielles de manière à isoler thermiquement.

35. Procédé selon l'une quelconque des six revendications précédentes, **caractérisé en ce que** les produits organiques séparés via la tête sont recyclés dans la transformation de DEG avec de l'ammoniac.

36. Procédé selon l'une quelconque des revendications précédentes pour la séparation de mélanges contenant de la morpholine (MO), du monoaminodiglycol (ADG), de l'ammoniac, de l'eau, de la N-éthylmorpholine (E-MO), de la 1,2-éthylènediamine (EDA) et des produits organiques présentant un point d'ébullition > 224,8°C (1,013 bars).

37. Procédé selon l'une quelconque des revendications précédentes, **caractérisé en ce que** le mélange contenant la morpholine (MO), le monoaminodiglycol (ADG), l'ammoniac et l'eau a été obtenu par transformation de diéthylèneglycol (DEG) avec de l'ammoniac en présence d'un catalyseur hétérogène, contenant Cu, Ni et Co sur du dioxyde de zirconium comme support ou en présence d'un catalyseur hétérogène, contenant Cu et Ni sur de l'oxyde d'aluminium comme support.

38. Procédé selon l'une quelconque des revendications précédentes, **caractérisé en ce que** la chaleur produite par les condensats de buées et/ou la chaleur de réaction produite par la synthèse du mélange utilisé pour la séparation par distillation est utilisée par intégration dans les évaporateurs des colonnes K20, K30, K40, K50, K55 et/ou K60.

39. Procédé selon l'une quelconque des revendications précédentes pour la préparation de morpholine (MO) avec une pureté ≥ 99,5% en poids, une teneur en N-éthylmorpholine (E-MO) ≤ 0,20% en poids, une teneur en 1,2-éthylènediamine (EDA) ≤ 0,30% en poids, une teneur en 2-méthoxyéthanol < 0,50% en poids et une teneur en eau ≤ 0,05% en poids.

40. Procédé selon l'une quelconque des revendications précédentes pour la préparation de morpholine (MO) avec un indice de couleur ALPHA ≤ 10 et une teneur en chlorure ≤ 15 mg/litre.

41. Procédé selon l'une quelconque des revendications 22 à 40 pour la préparation de monoaminodiglycol (ADG) avec une pureté ≥ 98,00% en poids, une teneur en DEG ≤ 0,40% en poids, une teneur en eau ≤ 0,20% en poids et un indice de couleur APHA ≤ 20.

42. Procédé selon l'une quelconque des revendications 17 à 41 pour la préparation de N-éthylmorpholine (E-MO) avec une pureté ≥ 98,50% en poids, une teneur en eau ≤ 0,30% en poids et un indice de couleur APHA ≤ 50.
